(19) Europäisches Patentamt
European Patent Office
Office européen des brevets

(11) EP 1 278 565 B1

(12) **FASCICULE DE BREVET EUROPEEN**

(45) Date de publication et mention de la délivrance du brevet:
**12.07.2006 Bulletin 2006/28**

(21) Numéro de dépôt: **01919574.2**

(22) Date de dépôt: **27.03.2001**

(51) Int Cl.:
***A61M 5/30*** (2006.01)

(86) Numéro de dépôt international:
**PCT/FR2001/000921**

(87) Numéro de publication internationale:
**WO 2001/078810 (25.10.2001 Gazette 2001/43)**

(54) **SERINGUE SANS AIGUILLE FONCTIONNANT AVEC UN CHARGEMENT PYROTECHNIQUE BICOMPOSITION**

NADELLOSE SPRITZE MIT EINER PYROTECHNISCHEN ZWEIKOMPONENTENLADUNG

NEEDLELESS SYRINGE FUNCTIONING WITH A DOUBLE-COMPOSITION PYROTECHNIC CHARGE

(84) Etats contractants désignés:
**AT BE CH CY DE DK ES FI FR GB GR IE IT LI LU MC NL PT SE TR**

(30) Priorité: **19.04.2000 FR 0005031**

(43) Date de publication de la demande:
**29.01.2003 Bulletin 2003/05**

(73) Titulaire: **Crossject**
**75181 Paris**
**Cédex 04 (FR)**

(72) Inventeurs:
- **ALEXANDRE, Patrick**
**F-70100 Gray (FR)**
- **COGNOT, Patrick**
**77350 Le Mee sur Seine (FR)**
- **LAFFORGUE, Joel**
**91760-Itteville (FR)**
- **ROLLER, Denis**
**F-91590 La Ferte Alais (FR)**

(74) Mandataire: **Maureau, Philippe et al**
**Cabinet GERMAIN & MAUREAU**
**39, rue de Liège**
**75008 Paris (FR)**

(56) Documents cités:
**WO-A-98/31409** **WO-A-99/22790**
**FR-A- 2 774 684** **US-A- 3 802 430**
**US-A- 4 059 107** **US-A- 4 124 024**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 1 278 565 B1

## Description

Le domaine technique de l'invention est celui des seringues sans aiguille pré-remplies et jetables, fonctionnant avec un générateur de gaz, et utilisées pour les injections intradermiques, sous-cutanées et intramusculaires, de principe actif liquide à usage thérapeutique en médecine humaine ou vétérinaire.

Pour les dispositifs d'injection selon l'invention, un principe actif liquide est constitué par un liquide plus ou moins visqueux, ou un mélange de liquide, ou un gel. Le principe actif peut être un solide mis en solution dans un solvant approprié pour l'injection. Il peut également être représenté par un solide pulvérulent mis en suspension plus ou moins concentré dans un liquide approprié. La granulométrie du principe doit être compatible avec le diamètre des conduits pour éviter les bouchages.

Les seringues sans aiguille selon l'invention ont la particularité de fonctionner avec un générateur de gaz pyrotechnique impliquant une charge pyrotechnique constituée par le mélange de deux poudres dont le principal intérêt est de maîtriser, au cours du temps, la pression du principe actif liquide en sortie de buse, de façon à ce que chaque phase de l'injection soit effectuée dans les conditions requises.

Il s'avère que dans le domaine des seringues sans aiguille pour l'injection de principe actif liquide, aucun brevet ne se rapporte à la mise en oeuvre d'un générateur de gaz pyrotechnique faisant intervenir le mélange de deux poudres. En revanche, l'utilisation d'une charge pyrotechnique simple pour ce type de seringue existe déjà et a fait l'objet de plusieurs brevets. A titre d'exemple, on peut citer le brevet US 2,322,244 relatif à un injecteur hypodermique sans aiguille fonctionnant à partir d'une cartouche à blanc. Le liquide à injecter, étant placé au contact de la cartouche, est expulsé de l'injecteur sous l'effet de la pression générée par les gaz de combustion. Un autre brevet, le WO 98/31409, décrit un système d'injection hypodermique impliquant une charge pyrotechnique constituée d'un explosif ou d'une poudre voir également le document US-A-4059107. La spécificité de cet injecteur est qu'il est conçu pour tenter de régler les problèmes liés à la cinétique d'expulsion du principe actif liquide, non pas en jouant sur les caractéristiques de la composition pyrotechnique, mais en présentant une géométrie particulière définissant notamment une chambre annexe d'expansion des gaz munie d'un évent. La charge pyrotechnique, qui se trouve à proximité immédiate du principe actif liquide, agit directement et instantanément sur ledit principe en lui communiquant une vitesse initiale très élevée, tandis que les gaz envahissent la chambre principale et la chambre annexe. La pression exercée sur le principe actif décroît alors pour venir se fixer à une valeur à peu près constante, suffisante pour le faire pénétrer dans la peau du patient. La chambre annexe permet de réguler cette pression. Enfin, le brevet US 2,704,542 se rapporte à une méthode d'injection par jet liquide. Cette méthode ne fait pas spécifiquement intervenir une charge pyrotechnique, mais implique un dispositif destiné à maîtriser les profils de pression. En l'occurrence, le procédé mis en place pour atteindre cet objectif réside dans le coulissement, en deux temps, d'un piston en deux parties constitué par un cylindre central de faible section logé dans un cylindre creux. Une pression amont provoque d'abord un déplacement de faible amplitude du cylindre central pour communiquer une impulsion brève mais très intense sur le liquide à expulser, puis l'ensemble du piston se déplace pour continuer à expulser ledit liquide, à la pression idoine, pour assurer une bonne pénétration.

Les seringues sans aiguille selon l'invention sont conçues pour assurer la pénétration, à travers la peau, de la totalité du principe actif liquide sans occasionner de déperditions dudit liquide pour insuffisance de vitesse, lesdites déperditions pouvant s'avérer préjudiciables pour la qualité de l'injection. Le procédé retenu pour parvenir à maîtriser la pression du liquide, en fonction du temps, en sortie de buse, consiste à utiliser une charge pyrotechnique constituée par le mélange de deux poudres, l'une dite « vive » et l'autre dite « lente », les caractéristiques dimensionnelles et chimiques de ces deux poudres étant conditionnées par la géométrie et les dimensions des seringues, ainsi que par le système d'injection incluant le réservoir de principe actif liquide, éventuellement un piston de poussée dudit principe actif et une buse comportant des orifices d'expulsion. La poudre vive, lorsqu'elle est mise en combustion, a pour fonction essentielle de communiquer quasi-instantanément au principe actif liquide un niveau de pression tel qu'il acquiert instantanément une vitesse de plusieurs centaines de mètres par seconde, lui permettant de pénétrer la peau du patient dès son expulsion de la seringue. La poudre lente, mise en combustion simultanément, permet de garantir au principe actif un niveau de pression minimum pendant toute la durée de l'injection, suffisant pour poursuivre la diffusion à travers l'orifice de la peau créé par l'effet de la poudre vive. Les notions de poudre « vive » et « lente » seront explicitées par la suite.

Ainsi, les seringues sans aiguille selon l'invention permettent, tout en conservant leur géométrie et leur encombrement réduit, d'assurer une injection fiable et propre, contrairement aux dispositifs d'injection décrits dans l'Etat de la Technique, et pour lesquels la recherche d'un profil de pression optimisé passe par une modification de leur structure, illustrée par l'ajout de pièces supplémentaires ou de volumes annexes, accroissant ainsi leur encombrement et rendant plus complexe leur mécanisme de fonctionnement.

De plus, quelle que soit la configuration de la seringue pouvant être dictée par des impératifs liés à la spécificité d'une injection, il est toujours possible de déterminer un mélange de poudres adapté pour assurer une injection satisfaisante, sans rien avoir à modifier sur ladite seringue. En effet, le principe actif liquide peut se présenter en plus ou moins grande quantité, sous forme plus ou moins visqueuse, dans une seringue à architecture linéaire ou compacte.

Le mélange de poudre sera défini en tenant compte de toutes ces contraintes.

Les seringues sans aiguille selon l'invention assurent une injection propre et fiable et autorisent une très grande souplesse d'utilisation de par la grande variabilité des compositions pyrotechniques pouvant être retenues pour le mélange et ce, en s'affranchissant de tout encombrement supplémentaire.

L'objet de l'invention concerne une seringue sans aiguille comprenant successivement un générateur de gaz pyrotechnique, au moins un piston, une réserve de principe actif liquide et une buse d'éjection, caractérisée en ce que le générateur de gaz pyrotechnique comprend une charge pyrotechnique constituée par le mélange d'au moins deux poudres.

Préférentiellement, la charge pyrotechnique est constituée par le mélange d'une première poudre et d'une deuxième poudre.

Les poudres se caractérisent, d'une part, par leur formulation chimique et, d'autre part, par leur géométrie. La formulation chimique intègre tous les composants intervenant dans la poudre et auxquels il faut adjoindre un coefficient pondérateur correspondant à la fraction massique dudit composant. La géométrie de la poudre se ramène à la géométrie de chaque grain la constituant. Un grain se définit par sa forme, ses dimensions et le nombre de trous qu'il possède, lesdits trous contribuant à déterminer une épaisseur à brûler.

Lorsqu'il est précisé que la charge pyrotechnique est constituée par le mélange d'une première poudre et d'une deuxième poudre, cela signifie que les deux poudres sont différentes l'une de l'autre et que cette différence peut ne porter que sur un seul des paramètres évoqués ci-avant. Autrement dit, les deux poudres peuvent, par exemple, avoir la même composition chimique mais présenter des grains de géométrie légèrement différente.

Avantageusement, la charge pyrotechnique consiste en un mélange de deux poudres en vrac, c'est à dire que les deux poudres se présentent à l'état de grains qui sont mélangés aléatoirement, sans ordonnancement particulier, la poudre résultante épousant la forme du conteneur dans lequel elle se trouve, tout en ménageant des interstices entre les grains. Mais il peut également être envisagé qu'au moins l'une des deux poudres se présente de façon ordonnée ou particulière, comme, par exemple, sous la forme d'un fagot de brins ou sous la forme d'un grain unique de taille importante, voire sous forme agglomérée.

Selon une autre variante préférée de l'invention, la charge pyrotechnique est constituée par le mélange de deux poudres se présentent chacune sous forme d'un bloc compact, lesdits blocs pouvant, soit se retrouver au contact et en continuité l'une de l'autre, soit être concentriques pour ne définir qu'un seul bloc ayant dans sa partie centrale la composition de la première poudre et en partie périphérique la composition de la deuxième poudre, ou vice versa selon l'allumage.

De façon préférentielle, la première poudre a une vivacité dynamique supérieure à 8 $(\text{MPa.s})^{-1}$.

De façon avantageuse, la deuxième poudre a une vivacité dynamique inférieure à 16 $(\text{MPa}\cdot\text{s})^{-1}$ et qui est systématiquement inférieure à celle de la première poudre.

En fait, il s'agit de la valeur de la vivacité dynamique d'un grain de poudre, à mi-combustion. La vivacité dynamique est un paramètre qui traduit la vivacité d'une poudre tout au long de la combustion.

Elle est donnée par la formule :

$$L(z) = \frac{1}{P} \cdot \frac{1}{P_{max}} \left(\frac{dP}{dt}\right)$$

où P est la pression instantanée correspondant à l'état d'avancement z.

Pmax est la pression maximale atteinte.

$\frac{dP}{dt}$ est la dérivée de la pression au cours du temps

$$z = \frac{P}{P_{max}} .$$

Les conditions dans lesquelles ont été obtenues les valeurs de la vivacité dynamique sont les suivantes :

- il s'agit de la valeur de la vivacité dynamique à la mi-combustion, c'est à dire la valeur correspondant à z = 0, 5,
- les valeurs ont été obtenues pour des tirs en enceinte manometrique ayant un volume de chambre de 27,8 $cm^3$,

- la densité de chargement est de 0,036 g/cm$^3$,
- la masse de poudre est de 1 g.

Pour les seringues sans aiguille selon l'invention, la charge pyrotechnique est constituée par le mélange d'une poudre de vivacité élevée avec une poudre de vivacité plus faible, d'où la dénomination «poudre vive » et « poudre lente ». La poudre de vivacité élevée assure une montée en pression rapide de l'ordre de 1 ms, tandis que la poudre de vivacité faible permet de poursuivre la production de gaz pendant l'injection, afin de compenser la baisse de pression due à l'augmentation du volume de la chambre de combustion causée par le déplacement du piston, et de compenser également les pertes thermiques aux parois, pendant 4 à 8 ms. L'utilisation de deux poudres de vivacité différente entraîne, par ailleurs, une diminution de la pression maximale de fonctionnement, permettant de réduire la résistance mécanique du dispositif et donc les coûts de fabrication : en effet, si la charge pyrotechnique n'était constituée que d'une seule poudre vive, le profil de pression dans le principe actif liquide ressemblerait à celui d'une détente pure.

Pour que la pression de fin d'injection ne soit pas inférieure à la pression seuil d'injection en dessous de laquelle le liquide ne pénètre plus correctement dans les tissus, il faudrait augmenter la pression maximale afin de décaler vers le haut le profil précédent, de manière à ce que durant toute la durée de l'injection, la pression d'injection demeure toujours supérieure à la pression seuil. En utilisant un mélange de deux poudres de vivacité différente, il est possible de maintenir la pression d'injection au dessus de la valeur seuil sans pour autant avoir à augmenter la pression maximale.

La montée rapide en pression au début de l'injection est nécessaire pour assurer une bonne pénétration dans la peau sans fuite de principe actif.

De façon préférentielle, la poussée du principe actif liquide est assurée par un piston simple, transmettant au liquide la pression résidant dans la chambre d'expansion des gaz, en diminuant son intensité mais en conservant le profil général de sa variation en fonction du temps. De façon plus générale, la charge pyrotechnique peut s'adapter au nombre de pistons impliqués dans la poussée du principe actif liquide, à leur forme, à leur nature ainsi qu'à la géométrie de la buse et au nombre de trous qu'elle possède. En effet, une poudre se caractérisant par de nombreux paramètres chimiques et structurels, le mélange de deux poudres offre un nombre quasi-illimité de combinaisons pouvant répondre à toute sorte de situation.

De façon avantageuse, au moins l'une des deux poudres est à base de nitrocellulose dont le taux massique est compris entre 0,45 et 0,99. Le taux massique d'un composant se caractérise comme étant le rapport de la masse de ce composant sur la masse totale de tous les composants. Avantageusement, le taux massique de nitrocellulose est compris entre 0,93 et 0,98.

En effet, les nitrocelluloses, de par leurs propriétés spécifiques, représentent la base essentielle des poudres couramment utilisées pour la propulsion de balles, d'obus ou de projectiles divers dans les armes à tubes. Selon un premier mode de réalisation de l'invention, chaque poudre qui est à base de nitrocellulose contient aussi un ester nitrique, comme par exemple de la nitroglycérine. Préférentiellement, pour les poudres contenant ces deux composants, le taux massique de nitrocellulose est compris entre 0,49 et 0,61 et le taux massique de nitroglycérine est compris entre 0,35 et 0,49. Avantageusement, la première poudre est choisie parmi les poudres poreuses. De façon préférentielle, la première poudre qui est poreuse contient de la nitrocellulose et le taux massique de nitrocellulose est compris entre 0,93 et 0,98. Une poudre à base de nitrocellulose est rendue poreuse par l'incorporation, lors de la phase de malaxage de leur procédé de fabrication, d'un sel comme le nitrate de potassium qui est ensuite retiré par dissolution. Les cristaux de nitrate de potassium restés incorporés à la surface des grains de poudre, constituent des points chauds sous l'influence d'un allumeur. Une surface poreuse permet donc, entre autre, d'améliorer l'allumage de la poudre.

De façon avantageuse, la première poudre a une épaisseur à brûler inférieure ou égale à 0,5 mm. L'épaisseur à brûler correspond à la plus petite dimension du grain de poudre suivant laquelle le front de combustion va progresser puis s'arrêter, permettant ainsi de fixer le temps de combustion dudit grain. Comme un grain de poudre brûle par toutes ses faces à la fois, l'épaisseur à brûler correspond à la moitié de sa plus petite épaisseur. Cette épaisseur à brûler dépend de la forme du grain, de ses dimensions ainsi que du nombre et de la position des trous qu'il possède.

Les grains constitutifs des poudres pouvant être mélangées pour réaliser une charge pyrotechnique conforme à celle utilisée pour les seringues sans aiguille selon l'invention, peuvent revêtir diverses formes. Ils peuvent, par exemple, être monotubulaires, multitubulaires, sphériques, sphériques écrasés, cylindriques ou se retrouver sous forme de paillettes ou de bâtonnets. Pour chacune de ces géométries, l'épaisseur à brûler représente un paramètre parfaitement identifié. Par exemple,

- pour un grain sphérique, l'épaisseur à brûler correspond au rayon du grain,
- pour un grain cylindrique, de longueur importante, l'épaisseur à brûler correspond au rayon du grain,
- pour un grain monotubulaire, l'épaisseur à brûler correspond à la demi-épaisseur du grain pris selon une direction radiale,
- pour un grain multitubulaire pour lequel les trous sont régulièrement espacés entre eux, l'épaisseur à brûler correspond à la demi-longueur séparant deux trous successifs.

Il est particulièrement recommandé de choisir, comme poudre vive, une poudre ayant une faible épaisseur à brûler. Avantageusement, la poudre vive est poreuse et est à base de nitrocellulose. Avantageusement, elle possède une épaisseur à brûler égale à 0,3 mm et se présente sous forme de bâtonnets ou de paillettes.

Préféreritiellement, la première poudre a un temps de combustion inférieur à 6 millisecondes. Il s'agit d'un temps correspondant à une situation réelle, c'est à dire à une configuration « seringue » impliquant les conditions suivantes :

- il s'agit d'une combustion de poudre dans une chambre dont le volume final est de 1,6 $cm^3$,
- la poussée du liquide est assurée par une pièce constituée par un piston.

De façon préférentielle, la deuxième poudre possède une épaisseur à brûler comprise entre 0,1 mm et 1 mm.

De façon avantageuse, la deuxième poudre a un temps de combustion supérieur à 4ms et qui est systématiquement supérieur à celui de la première poudre. Le temps de combustion de la deuxième poudre a été obtenu dans les mêmes conditions que celles dans lesquelles a été déterminé celui de la première poudre. Le temps de combustion de la deuxième poudre doit toujours excéder celui de la première poudre, puisque la deuxième poudre ne doit sa présence dans le mélange que pour suppléer le manque de pression observée lors de la combustion de la seule première poudre. Les temps de combustion des deux poudres sont liés à la spécificité de l'injection et, notamment, à l'association entre le volume de principe actif à injecter et les caractéristiques de la buse portant essentiellement sur le nombre de canaux d'évacuation, leur répartition et leur diamètre. Selon un mode de réalisation préféré de l'invention, la masse totale des deux poudres est inférieure à 100 mg. Cette limite seuil est dictée, d'une part, par les impératifs liés à l'injection requérant notamment une vitesse du liquide à l'impact sur la peau comprise entre 100 m/s et 200 m/s, et, d'autre part, aux dimensions de la seringue sans aiguille qui doivent rester compatibles avec celles d'un objet de petite taille, léger et maniable. Avantageusement, le rapport de la masse de la première poudre sur la masse totale des deux poudres est supérieur à 0,1. En effet, les caractéristiques de l'impulsion initiale devant communiquer instantanément une vitesse très élevée au principe actif liquide, nécessite une quantité minimale de poudre qui ne peut être inférieure à 10% de la masse totale de poudre.

Selon une première variante préférée de l'invention, la fonction de forme de la deuxième poudre est progressive. En fait, la fonction de forme d'une poudre se ramène à la fonction de forme d'un grain la constituant en supposant tous les grains identiques. La fonction de forme d'un grain est donnée par le rapport S/So où So est la surface de combustion initiale du grain et S sa surface de combustion à un certain état d'avancement de ladite combustion. Cette fonction de forme traduit l'évolution de la surface de combustion d'un grain en fonction du temps au cours de la combustion. Pour une poudre donnée, plus la surface de combustion est importante, plus la quantité de gaz libérée par unité de temps est importante et plus la montée en pression dans un volume clos est rapide. Lorsque le piston se déplace en début d'injection, le volume de la chambre de combustion croît progressivement, et, dans l'optique de maintenir un niveau de pression sensiblement constant dans ledit volume croissant, il est souhaitable d'utiliser une deuxième poudre lente à fonction de forme progressive.

Selon une deuxième variante préférée de l'invention, la fonction de forme de la deuxième poudre est quasi constante. En effet, dans certaines conditions, et notamment suivant la nature de la première poudre vive retenue, une deuxième poudre lente ayant une fonction de forme constante peut suffire. La fonction de forme dépendant essentiellement de la géométrie du grain de poudre, les grains de la deuxième poudre lente auront donc préférentiellement une forme multitubulaire ou monotubulaire pour lesquels les fonctions de forme sont respectivement progressive et quasi constante.

Préférentiellement, les poudres multitubulaires auront trois trous sept trous ou dix-neuf trous selon le profil de pression recherché.

Avantageusement, le générateur de gaz pyrotechnique comprend un dispositif d'initiation de la charge pyrotechnique faisant intervenir un dispositif de percussion et une amorce. Il est également possible d'utiliser un système d'initiation à base d'un cristal piézo-électrique ou d'un rugueux.

Les seringues sans aiguilles selon l'invention présentent l'avantage de garantir une injection satisfaisante de la totalité du principe actif liquide, en conservant un mécanisme simple de fonctionnement ainsi qu'un encombrement réduit ne nécessitant, ni la mise en place de pièces spécifiques, sources d'usinages et de coûts supplémentaires, ni une modification en profondeur de la géométrie du corps desdites seringues.

De plus, la grande variabilité des compositions pyrotechniques pouvant être retenues pour les mélanges, permet d'obtenir une très grande variété de profils de pression aptes à s'adapter à toutes les configurations possibles. Enfin, la parfaite maîtrise des effets engendrés par la combustion d'une charge pyrotechnique associée à des systèmes d'allumage largement éprouvés, confère aux seringues sans aiguille selon l'invention un caractère de grande fiabilité et de sûreté.

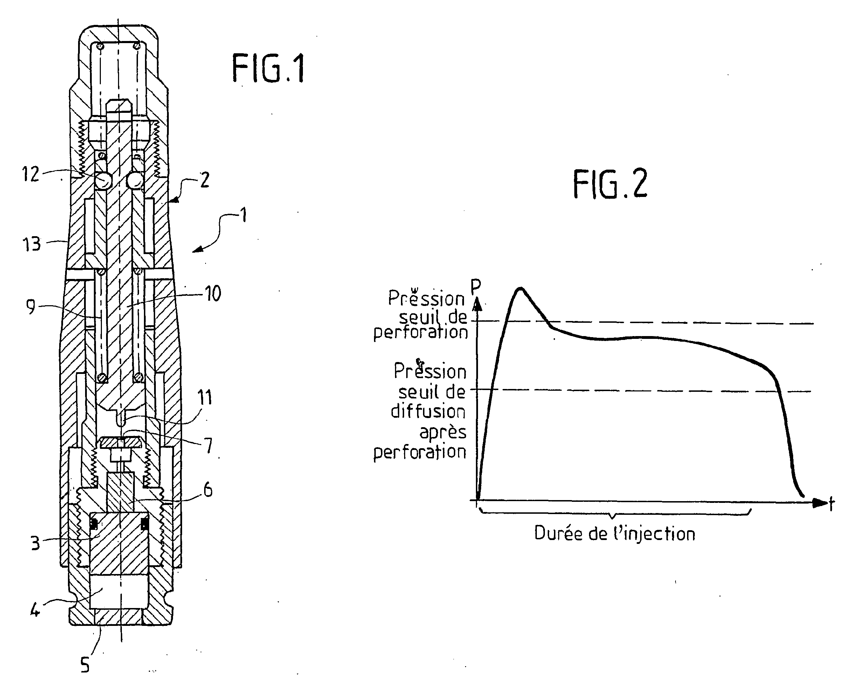

Les exemples non limitatifs suivants illustrent l'invention en se référant aux figures 1 et 2.

La figure 1 est une vue en coupe axiale longitudinale d'une seringue sans aiguille selon l'invention.

La figure 2 est un graphe simplifié typique de la variation de pression dans le liquide en fonction du temps, engendrée par la combustion d'un chargement bicomposition dans une seringue selon l'invention.

En se référant à la figure 1, une seringue sans aiguille 1 selon l'invention comprend un générateur de gaz pyrotechnique 2, un piston 3, un réservoir de principe actif liquide 4 et une buse d'éjection 5. Les termes « buse d'injection » et « buse d'éjection » sont équivalents.

Le générateur pyrotechnique 2 de gaz comprend un dispositif d'initiation d'une charge pyrotechnique 6 faisant intervenir un dispositif de percussion et une amorce 7. Le dispositif de percussion qui est déclenché par un bouton poussoir 8 comprend un ressort 9 précontraint et une masselotte 10 allongée munie d'un percuteur 11. La masselotte 10 est bloquée par au moins une bille 12 de maintien coincée entre ladite masselotte 10 et un corps cylindrique creux 13 dans lequel est susceptible de se déplacer ladite masselotte 10. L'amorce 7 et la charge pyrotechnique 6, de forme sensiblement cylindrique, sont logées dans le corps cylindrique creux 13, en aval de la masselotte 10. La charge pyrotechnique 6 débouche dans un espace élargi, de forme sensiblement cylindrique, occupé dans sa partie amont par le piston 3 et dans sa partie aval par le réservoir de principe actif liquide 4, ledit espace élargi étant obturé à son extrémité par la buse d'éjection 5 munie de plusieurs canaux permettant de mettre en communication le principe actif 4 et l'extérieur de la seringue 1. Ces différents éléments s'agencent entre eux de sorte qu'ils se présentent en continuité les uns des autres, la charge pyrotechnique 6 étant au contact du piston 3, lui-même au contact du principe actif liquide 4, lui-même délimité par la buse 5. Afin d'éviter au principe actif liquide 4 de s'échapper de la seringue 1, un bouchon est fixé au niveau de la buse 5 en obturant ses canaux, ledit bouchon étant retiré avant usage. La charge pyrotechnique 6 est constituée par le mélange de deux poudres en vrac.

Le mode de fonctionnement d'une seringue 1 sans aiguille selon l'invention s'effectue comme suit.

L'utilisateur positionne la seringue 1 de façon à ce que la buse 5 vienne en appui contre la peau du patient à traiter.

Une pression sur le bouton poussoir 8 permet au corps cylindrique creux 13 de se déplacer jusqu'à ce que sa partie évasée se présente en face de la bille 12 de maintien. La bille 12 sort de son logement, libérant alors la masselotte 10, qui, soumise à l'action du ressort 9 qui se détend est brutalement accélérée vers l'amorce 7, le percuteur 11 en avant. La réaction de l'amorce 7 entraîne la mise à feu de la charge pyrotechnique 6 qui se décompose en émettant des gaz.

En se référant à la figure 2, la poudre vive communique alors au piston 3 une vitesse initiale de déplacement élevée de façon à ce que le principe actif liquide 4, dès sa sortie de la buse 5, puisse immédiatement être animé d'une vitesse suffisamment élevée pour pénétrer la peau. La poudre lente maintient dans le principe actif liquide 4 un niveau de pression seuil lui permettant, pour la suite de l'injection, de préserver son pouvoir de diffusion à travers la peau, une fois celle-ci perforée. De cette manière, l'injection s'effectue proprement sans aucune déperdition de principe actif liquide 4.

Les exemples non limitatifs suivants illustrent la caractéristique essentielle de l'invention qui se rapporte à la charge pyrotechnique 6.

Exemple 1 :

Les tableaux ci-après récapitulent les principales caractéristiques des deux poudres ayant servi pour le premier mélange.

I - Composition chimique

Poudre vive

| COMPOSANTS | Fraction massique x 100 |
|---|---|
| Nitrocellulose | 93,0 |
| Dinitrotoluène | 2,0 |
| Dibutylphtalate | 1,2 |
| Diphénylamine | 1,0 |
| Graphite | 0,5 |
| Solvant résiduel | 0,2 |
| Sel résiduel | 0,4 |
| Humidité | 1,2 |
| Colorant | Traces |

Poudre lente

| COMPOSANTS | Fraction massique x 100 |
|---|---|
| Nitrocellulose | 95 |
| Additifs | 5 |

II - Caractéristiques structurelles et paramètres liés à la combustion

| | Porosité | Temps de combustion (ms) | Vivacité dynamique à mi-combustion $(MPa.s)^{-1}$ | Epaisseur à brûler (mm) | Forme des grains | Fonction de forme |
|---|---|---|---|---|---|---|
| Poudre vive | OUI | 0,8 | 24 | 0,2-0,5 | paillette | dégressive |
| Poudre lente | NON | 3,1 | 11 | 0,22 | mono-tubulaire | quasi constante |

Le volume du principe actif liquide à injecter est de 0,5 ml. Les quantités de poudres ont alors été déterminées en fonction des caractéristiques de la buse et notamment du nombre de ses canaux d'injection. Les valeurs de diamètre données ci-après correspondent à des diamètres équivalents. En effet, dans la réalité, les canaux sont des rainures longitudinales hemi-cylindriques dont le diamètre réel est de 350 µm. Si l'on assimile les canaux a de parfaits cylindres de section identique, alors il faut faire état d'un diamètre équivalent de 250 µm. Les diamètres mentionnés ci-après sont donc des diamètres équivalents.

Buse à 3 canaux de diamètre 250 $\mu$m

| | |
|---|---|
| Poudre vive : | 30 mg |
| Poudre lente : | 30 mg. |

Buse à 6 canaux de diamètre 250 $\mu$m

| | |
|---|---|
| Poudre vive : | 31 mg |
| Poudre lente : | 25 mg. |

Lorsque le nombre de canaux diminue, la durée de l'injection augmente. Le rapport poudre lente/poudre vive doit donc augmenter afin de garder une pression de fin d'injection suffisante. Lorsque la durée d'injection augmente, la masse totale de poudre doit augmenter pour limiter l'effet des pertes thermiques, mais par ailleurs, l'efficacité de la poussée, en termes de % et profondeur de pénétration, sera d'autant meilleure que le nombre de canaux est plus réduit, ce qui va dans le sens d'une limitation de la masse nécessaire de poudre.

Pour cet exemple, ces quantités de poudres correspondent aux charges minimales permettant d'obtenir un pourcentage de pénétration voisin de 99% avec une profondeur de pénétration de 12 à 15 mm et ce, en réduisant notablement la pression maximale dans le liquide de la seringue.

Exemple 2 :

Les principales caractéristiques des deux poudres utilisées pour le deuxième mélange sont résumées dans le tableau ci-après :

I - Composition chimique

| | COMPOSANTS | Fraction massique x 100 |
|---|---|---|
| Poudre vive | Nitrocellulose | 93,0 |
| | Dinitrotoluène | 2,0 |
| | Dibutylphtalate | 1,2 |
| | Diphénylamine | 1,0 |
| | Graphite | 0,5 |
| | Solvant résiduel | 0,2 |
| | Sel résiduel | 0,4 |
| | Humidité | 1,2 |
| | Colorant | Traces |

Poudre lente

| COMPOSANTS | Fraction massique x 100 |
|---|---|
| Nitrocellulose | 95 |
| Additifs | 5 |

II - Caractéristique structurelles et paramètres liés à la combustion

| | Porosité | Temps de combustion (ms) | Vivacité dynamique à mi-combustion $(MPa.s)^{-1}$ | Epaisseur à brûler (mm) | Forme des grains | Fonction de forme |
|---|---|---|---|---|---|---|
| Poudre vive | OUI | 0,8 | 24 | 0,2-0,5 | paillette | dégressive |
| Poudre lente | NON | 6 | 6 | 0,51 | hepta-tubulaire | progressive |

Pour une buse à 6 canaux de diamètre 250 µm, les quantités de poudres retenues sont :

Poudre vive : 42,5 mg
Poudre lente : 23,5 mg.

Ces quantités de poudres permettent d'obtenir un pourcentage de pénétration supérieur à 99%.
En adaptant le chargement pyrotechnique à la buse, il est possible d'obtenir un profil de pression dans le liquide en trois phases.
La phase initiale de montée en pression, qui doit être rapide, est obtenue avec une poudre vive.
L'utilisation d'une poudre ayant une épaisseur à brûler importante, adaptée, permet au cours de la deuxième phase, de compenser par le débit gazeux, la baisse de pression due à l'augmentation du volume de la chambre de combustion et aux pertes thermiques.
Enfin, la troisième phase qui correspond à la simple détente du gaz de combustion jusqu'à la fin de l'injection, ne nuit pas à la qualité de l'injection. Elle est même souhaitable pour limiter la profondeur de pénétration des jets dans la peau.
Avec un chargement pyrotechnique ainsi adapté, on injecte 0,5 ml de principe actif liquide dans de bonnes conditions.

## Revendications

1. seringue sans aiguille comprenant successivement un générateur de gaz pyrotechnique (2), au moins un piston (3), une réserve de principe actif liquide (4) et une buse d'éjection (5), **caractérisée en ce que** le dit générateur (2) possédant une charge pyrotechnique (6) constituée par le mélange d'une première poudre avec une deuxième poudre, la première poudre ayant une vivacité dynamique élevée $(MPa.s)^{-1}$ et la deuxième poudre ayant une vivacité dynamique plus faible $(MPa.s)^{-1}$.

2. Seringue sans aiguille selon la revendication 1, **caractérisée en ce que** la première poudre a une vivacité dynamique supérieure à 8 $(MPa.s)^{-1}$.

3. Seringue sans aiguille selon la revendication 1, **caractérisée en ce que** la deuxième poudre a une vivacité dynamique inférieure à 16 $(MPa.s)^{-1}$.

4. Seringue sans aiguille selon l'une quelconque des revendications 1, 2, 3, **caractérisée en ce que** au moins l'une des deux poudres est à base de nitrocellulose dont le taux massique est compris entre 0.45 et 0.99.

5. Seringue sans aiguille selon la revendication 4, **caractérisée en ce que** chaque poudre qui est à base de nitrocellulose contient aussi de la nitroglycérine.

6. Seringue sans aiguille selon l'une quelconque des revendications 1, 2 ou 4, **caractérisée en ce que** la première poudre est choisie parmi les poudres poreuses.

7. Seringue sans aiguille selon la revendication 2, **caractérisée en ce que** la première poudre a une épaisseur à brûler inférieure ou égale à 0.5 mm.

8. Seringue sans aiguille selon la revendication 2, **caractérisée en ce que** la première poudre a un temps de combustion inférieur à 6 ms.

9. Seringue sans aiguille selon la revendication 3, **caractérisée en ce que** la deuxième poudre possède une épaisseur à brûler comprise entre 0.1 mm et 1 mm.

10. Seringue sans aiguille selon la revendication 3, **caractérisée en ce que** la deuxième poudre a un temps de combustion supérieur à 4 ms et qui est systématiquement supérieur à celui de la première poudre.

11. Seringue sans aiguille selon la revendication 1, **caractérisée en ce que** la masse totale des deux **poudres est inférieure à 100 mg.**

12. Seringue sans aiguille selon la revendication 2, **caractérisée en ce que** le rapport de la masse de la première poudre sur la masse totale des deux poudres est supérieur à 0.1.

13. Seringue sans aiguille selon la revendication 3, **caractérisée en ce que** la fonction de forme de la deuxième est progressive.

14. **Seringue sans aiguille selon la revendication 3, caractérisée en ce que** la fonction de forme de la deuxième poudre est quasi constante.

15. Seringue sans aiguille selon la revendication 1, **caractérisée en ce que** le générateur de gaz pyrotechnique (2) comprend un dispositif d'initiation de la charge pyrotechnique (6), faisant intervenir un dispositif de percussion et une amorce (7).

**Claims**

1. Needleless syringe comprising in succession a pyrotechnic gas generator (2), at least one piston (3), a reservoir of liquid active ingredient (4) and an injection nozzle (5), **characterised in that** the said generator (2) has a pyrotechnic charge (6) constituted by the mixture of a first powder with a second powder, the first powder having high dynamic liveliness $(MPa \cdot s)^{-1}$, and the second powder having lower dynamic liveliness $(MPa \cdot s)^{-1}$.

2. Needleless syringe according to claim 1, **characterised in that** the first powder has dynamic liveliness which is greater than 8 $(MPa.s)^{-1}$.

3. Needleless syringe according to claim 1, **characterised in that** the second powder has dynamic liveliness which is lower than 16 $(MPa \cdot s)^{-1}$.

4. Needleless syringe according to any one of claims 1, 2 or 3, **characterised in that** at least one of the two powders is based on nitrocellulose, the mass proportion of which is between 0.45 and 0.99.

5. Needleless syringe according to claim 4, **characterised in that** each powder which is based on nitrocellulose also contains nitroglycerine.

6. Needleless syringe according to any one of claims 1, 2 or 4, **characterised in that** the first powder is selected from amongst porous powders.

7. Needleless syringe according to claim 2, **characterised in that** the first powder has a burning thickness which is 0.5 mm or less.

8. Needleless syringe according to claim 2, **characterised in that** the first powder has a combustion time of less than 6 ms.

9. Needleless syringe according to claim 3, **characterised in that** the second powder has a burning thickness of between 0.1 mm and 1 mm.

10. Needleless syringe according to claim 3, **characterised in that** the second powder has a combustion time of more than 4 ms, and which is systematically greater than that of the first powder.

11. Needleless syringe according to claim 1, **characterised in that** the total weight of the two powders is less than 100 mg.

12. Needleless syringe according to claim 2, **characterised in that** the ratio of the weight of the first powder to the total weight of the two powders is greater than 0.1.

13. Needleless syringe according to claim 3, **characterised in that** the function of form of the second is progressive.

14. Needleless syringe according to claim 3, **characterised in that** the function of form of the second powder is virtually constant.

15. Needleless syringe according to claim 1, **characterised in that** the pyrotechnic gas generator (2) comprises a device for initiation of the pyrotechnic charge (6) which makes a percussion device and a primer (7) intervene.

**Patentansprüche**

1. Spritze ohne Nadel, welche nacheinander einen pyrotechnischen Gasgenerator (2), wenigstens einen Kolben (3), einen Vorrat an flüssigem Wirkstoff (4) und eine Ejektordüse (5) umfasst, **dadurch gekennzeichnet, dass** der Generator (2) eine pyrotechnische Ladung (6) besitzt, die von dem Gemisch eines ersten Pulvers mit einem zweiten Pulver gebildet wird, wobei das erste Pulver eine hohe dynamische Lebhaftigkeit $(MPa\cdot s)^{-1}$ aufweist und das zweite Pulver eine geringere dynamische Lebhaftigkeit $(MPa.s)^{-1}$ aufweist.

2. Spritze ohne Nadel nach Anspruch 1, **dadurch gekennzeichnet, dass** das erste Pulver eine dynamische Lebhaftigkeit aufweist, die größer als 8 $(MPa\cdot s)^{-1}$ ist.

3. Spritze ohne Nadel nach Anspruch 1, **dadurch gekennzeichnet, dass** das zweite Pulver eine dynamische Lebhaftigkeit aufweist, die kleiner als 16 $(MPa\cdot s)^{-1}$ ist.

4. Spritze ohne Nadel nach einem der Ansprüche 1, 2, 3, **dadurch gekennzeichnet, dass** wenigstens eines der zwei Pulver ein Pulver auf der Basis von Cellulosenitrat ist, dessen Massenanteil zwischen 0,45 und 0,99 beträgt.

5. Spritze ohne Nadel nach Anspruch 4, **dadurch gekennzeichnet, dass** jedes Pulver, welches ein Pulver auf der Basis von Cellulosenitrat ist, auch Nitroglycerin enthält.

6. Spritze ohne Nadel nach einem der Ansprüche 1, 2 oder 4, **dadurch gekennzeichnet, dass** das erste Pulver aus den porösen Pulvern gewählt ist.

7. Spritze ohne Nadel nach Anspruch 2, **dadurch gekennzeichnet, dass** das erste Pulver eine Brenndicke aufweist, die kleiner oder gleich 0,5 mm ist.

8. Spritze ohne Nadel nach Anspruch 2, **dadurch gekennzeichnet, dass** das erste Pulver eine Verbrennungszeit aufweist, die kleiner als 6 ms ist.

9. Spritze ohne Nadel nach Anspruch 3, **dadurch gekennzeichnet, dass** das zweite Pulver eine Brenndicke besitzt, die zwischen 0,1 mm und 1 mm beträgt.

10. Spritze ohne Nadel nach Anspruch 3, **dadurch gekennzeichnet, dass** das zweite Pulver eine Verbrennungszeit aufweist, die größer als 4 ms ist und die systematisch größer als die des ersten Pulvers ist.

11. Spritze ohne Nadel nach Anspruch 1, **dadurch gekennzeichnet, dass** die Gesamtmasse der zwei Pulver kleiner als 100 mg ist.

12. Spritze ohne Nadel nach Anspruch 2, **dadurch gekennzeichnet, dass** das Verhältnis der Masse des ersten Pulvers zur Gesamtmasse der zwei Pulver größer als 0,1 ist.

13. Spritze ohne Nadel nach Anspruch 3, **dadurch gekennzeichnet, dass** die Formfunktion des zweiten Pulvers wachsend ist.

14. Spritze ohne Nadel nach Anspruch 3, **dadurch gekennzeichnet, dass** die Formfunktion des zweiten Pulvers quasi konstant ist.

15. Spritze ohne Nadel nach Anspruch 1, **dadurch gekennzeichnet, dass** der pyrotechnische Gasgenerator (2) eine Vorrichtung zur Initiierung der pyrotechnischen Ladung (6) umfasst, in der eine Schlagvorrichtung und ein Zünder (7) verwendet werden.

P
t
Pression seuil de perforation
Pression seuil de diffusion après perforation
Durée de l'injection

FIG.2

1
2
10
11
7
6
12
13
9
3
4
5

FIG.1